(19) Europäisches Patentamt

European Patent Office

Office européen des brevets

(11) **EP 1 621 129 A1**

(12) **EUROPEAN PATENT APPLICATION**

(43) Date of publication:
**01.02.2006 Bulletin 2006/05**

(51) Int Cl.:
*A61B 5/022* (2006.01)    *F04B 43/04* (2006.01)

(21) Application number: **05015805.4**

(22) Date of filing: **20.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **21.07.2004 JP 2004212843**

(71) Applicant: **Omron Healthcare Co., Ltd.**
**Kyoto 615-0084 (JP)**

(72) Inventors:
• **Sano, Yoshihiko**
 **Omron Healthcare Co., Ltd.**
 **Kyoto-shi**
 **Kyoto 615-0084 (JP)**
• **Karo, Hiromichi**
 **Omron Healthcare Co., Ltd.**
 **Kyoto-shi**
 **Kyoto 615-0084 (JP)**

• **Kishimoto, Hiroshi**
 **Omron Healthcare Co., Ltd.**
 **Kyoto-shi**
 **Kyoto 615-0084 (JP)**
• **Tanaka, Takahide**
 **Omron Healthcare Co., Ltd.**
 **Kyoto-shi**
 **Kyoto 615-0084 (JP)**
• **Eda, Kenji**
 **Omron Healthcare Co., Ltd.**
 **Kyoto-shi**
 **Kyoto 615-0084 (JP)**
• **Miyata, Kiichiro**
 **Omron Healthcare Co., Ltd.**
 **Kyoto-shi**
 **Kyoto 615-0084 (JP)**

(74) Representative: **Kilian, Helmut**
 **Wilhelms, Kilian & Partner**
 **Patentanwälte**
 **Eduard-Schmid-Strasse 2**
 **81541 München (DE)**

(54) **Air pump, living body pressurization air intake and exhaust device, and electronic sphygmomanometer**

(57) An air pump includes an actuator that includes an elastomer or a polymer expandable or contractible when a voltage is applied to the elastomer or polymer, and electrodes provided to apply the voltage to the elastomer or polymer, and a diaphragm deformed by a reciprocating motion of the actuator, whereby the air pump dispensing with a component such as a motor or a clutch for deforming the diaphragm and having a simple configuration can be realized.

FIG. 9A

TO BLADDER 101

**Description**

Background of the invention

Field of the invention

The present invention relates to an air pump. Specifically, the present invention relates to an air pump suitably use in, for example, an electronic sphygmomanometer.

Description of the related art

In measuring blood pressure by means of an ordinary home sphygmomanometer, an air bag (a cuff) is attached to a part of a human body, e.g., an upper arm, the cuff is expanded by applying pressure to the cuff from the air pump, an artery of the human body is compressed, and the blood pressure value is calculated from an pressure pulse wave which is obtained at that time.

Recently, demand for reducing a size of a sphygmomanometer main body has risen from viewpoints of portability and containability, and a reduction in a size of the air pump included in the sphygmomanometer is desired accordingly.

Examples of the air pump used in the conventional sphygmomanometer are as follows.

Japanese Patent No. 2551757 discloses a small-sized pump configured so that a diaphragm connected to a drive body decentered by rotation of an output shaft of a motor supplies and exhausts the air by periodically changing its volume according to rotation of a drive shaft.

Japanese Patent No. 3373558 discloses a small-sized pump device that includes an operating rod decentered in a diameter direction of a motor by rotation of an output shaft of the motor, and a diaphragm body contracted and expanded by a reciprocating motion of the operating rod and performing a pump operation.

However, since the volume of the diaphragm that generates pressure by changing a volume of an air chamber is changed by driving the motor, the pump structure employing the conventional motor has the following disadvantages.

(1) It is necessary to convert a rotational motion of the motor into a vertical and horizontal reciprocating motion, so that energy loss occurs during conversion of the rotational motion into the reciprocating motion.

(2) To convert the rotational motion into the reciprocating motion as stated in (1) above, components and a space for the components are required.

(3) A motor rotating sound and a component driving sound are generated by the conversion of the rotational motion into the reciprocating motion.

Further, examples of a diaphragm pump employing not the motor but an electrostrictive polymer actuator are as follows.

Japanese Patent Application Laid-Open No. 2003-013861 discloses a pump configured so that a diaphragm consisting of an electrostrictive actuator is attached to a casing, a pump chamber is formed between the diaphragm and the casing, and a volume of the pump chamber is changed using a stroke and a force generated in a contraction direction of the electrostrictive actuator that is the diaphragm.

Japanese Patent Application Laid-Open No. 2003-193979 discloses a diaphragmpump configured so that a diaphragm consisting of either a piezoelectric device or an electrostrictive polymer actuator is attached to a casing, a pump chamber is formed between the diaphragm and the casing, a volume of the pump chamber is changed using a stroke and a force generated in a contraction direction of the actuator that is the diaphragm, and back pressure application means is provided on a back surface of the diaphragm.

However, these conventional pumps have the following disadvantages.

(4) The diaphragm consisting of the electrostrictive polymer actuator has a larger deformation amount and a stronger stroke if the electrostrictive polymer actuator is larger in area. In other words, to obtain a volume change necessary for the pump, it is necessary to provide an electrostrictive polymer actuator having a large area to some extent. However, pressure generated when the volume of the diaphragm is changed is entirely applied to the diaphragm that is displaced. The pressure is proportional to the area of the diaphragm. Due to this, the electrostrictive polymer actuator needs to have a large area and a greater displacement generating force. Thus, the diaphragm of this type is not suited for the reduction in the size of the pump.

(5) The above-stated diaphragm has a structure in which the electrostrictive polymer actuator configured so that a plurality of layers of annular expandable portions and electrodes are alternately formed generally concentrically is bonded to a disk-like metal plate. To change the volume of the pump chamber, however, it is necessary to generate not only a distortion deformation force of the electrostrictive polymer actuator that compresses the actual air but also a considerably great displacement generating force for distortion-deforming the metal plate into a concave shape.

Summary of the invention

The present invention has been achieved in view of the conventional techniques. It is an object of the present invention to provide an air pump having a simple configuration. It is another object of the present invention to provide a compact sphygmomanometer that includes the air pump.

To achieve the above-mentioned object, an air pump according to the present invention comprising: an actuator including an elastomer or a polymer expandable or contractible when a voltage is applied to the elastomer or polymer, and electrodes provided to apply the voltage to the elastomer or polymer; and a diaphragm deformed by a reciprocating motion of the actuator.

Alternatively, there is provided an air pump comprising: an actuator including an elastomer or a polymer expandable or contractible when a voltage is applied to the elastomer or polymer, and electrodes provided to apply the voltage to the elastomer or polymer; one or a plurality of housing members; and a diaphragm a part of which is fixed to at least one of the housing members, wherein one end of the actuator is fixed to the at least one of the housing members, the diaphragm is pressurized and deformed by an other end of the actuator that moves according to an expansion or a contraction of the elastomer or polymer, and a volume of a pump chamber formed between the diaphragm and the at least one of the housing members is changed by a deformation of the diaphragm, thereby pressurizing a fluid intaked into the pump chamber and discharging the fluid to an outside.

As the elastomer or polymer expandable or contractible when a voltage is applied thereto, a matter referred to as a dielectric elastomer or electrostrictive polymer (high strain plastic having electric field response such as silicon resin, acrylic resin, or polyurethane) can be used. An electroactive polymer artificial muscle ("EPAM") is most preferably used as the expandable or contractible elastomer or polymer.

With this constitution, the air pump dispensing with a component such as a motor or a clutch for deforming the diaphragm and having a simple configuration can be realized.

Also, it is preferred that the diaphragm includes an abutment portion abutting the other end of the actuator.

With this constitution, a driving force according to the expansion and contraction motion (reciprocating motion) of the actuator directly deforms the diaphragm. The energy transmission loss can be, therefore, suppressed.

Also, it is preferred that a magnitude of the expansion or the contraction of the actuator is changed according to a magnitude of the voltage applied to the actuator.

Also, it is preferred that the voltage applied to the actuator or a frequency of the voltage is controlled, thereby controlling a discharge flow rate and a discharge pressure of the fluid discharged from the pump chamber.

With this constitution, the magnitude of the voltage applied to the actuator or the frequency of the voltage is controlled, whereby a magnitude of the stroke or an oscillation frequency for allowing the actuator to deform the diaphragm can be changed, and the discharge flow rate and the discharge pressure can be controlled without changing the structure of the air pump.

Also, it is preferred that an air pump according to the invention further comprising: an intake portion for intaking the fluid into the pump chamber from the outside; and a discharge portion for discharging the fluid from the pump chamber to the outside, wherein check valves are provided between the intake portion and the pump chamber, and between the pump chamber and the discharge portion, respectively.

With this constitution, the air can be supplied into the pump chamber and discharged from the pump chamber with a simple configuration.

Also, it is preferred that an air pump according to the invention further comprising: a control valve that controls a pressure of the fluid discharged to the outside, wherein a passage communicating with the control valve is provided halfway from the pump chamber to the discharge portion.

With this constitution, the fluid pressurized by the diaphragm is controlled by the control valve, whereby the fluid can be discharged to the outside from the passage communicating with the control valve and the air pump capable of controlling reduction of pressure can be realized. Particularly by adopting the solenoid valve as the control valve, the small-sized air pump having a simple configuration can be realized.

There is provided a living body pressurization air intake and exhaust device comprising: a fluid bag wound around a living body and filled with a fluid such as the air; a cuff for fixing the fluid bag from surroundings of the fluid bag; and the air pump, for supplying the fluid to the fluid bag and pressurizing the fluid bag, which can preferably adopt the air pump according to the present invention.

With this constitution, the living body pressurization air intake and exhaust device small in size and light in weight and having a simple configuration can be realized.

There is provided an electronic sphygmomanometer comprising: a fluid bag wound around a living body and filled with a fluid such as the air; a cuff for fixing the fluid bag from surroundings of the fluid bag; the air pump, for supplying the fluid to the fluid bag and pressurizing the fluid bag; a pressure sensor that detects an internal air pressure of the fluid bag; and arithmetic means for executing a processing for measurement of blood pressure based on the detected internal air pressure, which can preferably adopt the air pump according to the present invention.

With this constitution, the electronic sphygmomanometer small in size and light in weight and having a simple configuration can be realized.

According to the present invention, the air pump having the simple configuration can be realized. In addition, the living body pressurization air intake and exhaust device and the electronic sphygmomanometer small in size and light in weight can be realized.

Brief Description of the Drawings

Figs. 1A and 1B are schematic sectional views that respectively depict operating states of an air pump according to a first embodiment;

Fig. 2 is an explodedperspective view of a diaphragm air pump according to the present embodiment;

Fig. 3 is an exploded perspective view of the diaphragm air pump according to the present embodiment;

Figs. 4A and 4B are sectional views that depict an intake state and a contracted state of the diaphragm air pump, respectively;

Figs. 5A and 5B are typical views that respectively depict deformation states if a voltage is applied to the actuator according to the present embodiment;

Figs. 6A and 6B are typical views for explaining a structure of an EPAM according to the present embodiment;

Fig. 7 is a block diagram that depicts a hardware configuration of an electronic sphygmomanometer according to a second embodiment;

Fig. 8 is a flowchart that depicts a basic operation of the electronic sphygmomanometer according to the second embodiment;

Figs. 9A and 9B are schematic sectional views that respectively depict operating states of an air pump according to the second embodiment; and

Fig. 10 is a schematic sectional view that depicts an operating state of the air pump according to the second embodiment.

Detailed description of the preferred embodiments

Most preferred embodiments of the present invention will be described hereinafter in detail with reference to the drawings. It shouldbenoted, however, that sizes, materials, shapes, functions, relative positions, etc. of constituent elements described in the embodiments are not intended to limit the scope of the present invention unless specified otherwise. Further, sizes, materials, shapes, functions, etc. of the members described once in the following explanation are the same as those explained initially unless specified otherwise.

(OUTLINE OF DIAPHRAGM AIR PUMP)

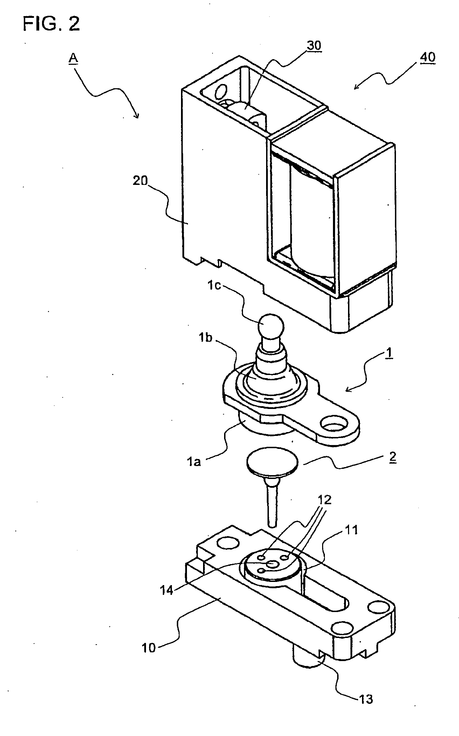

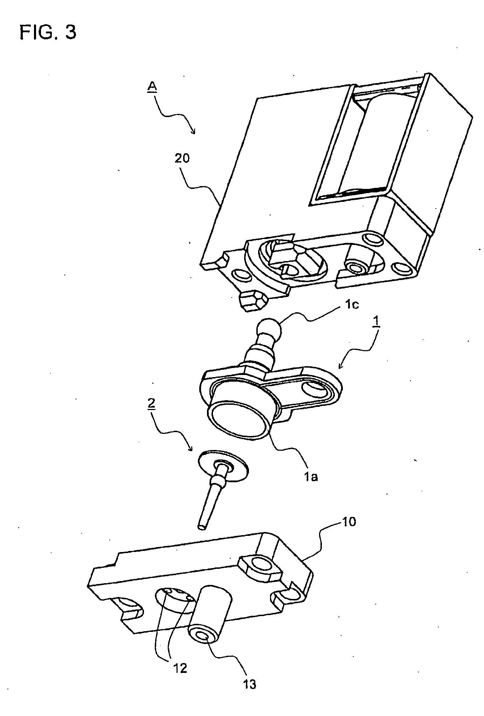

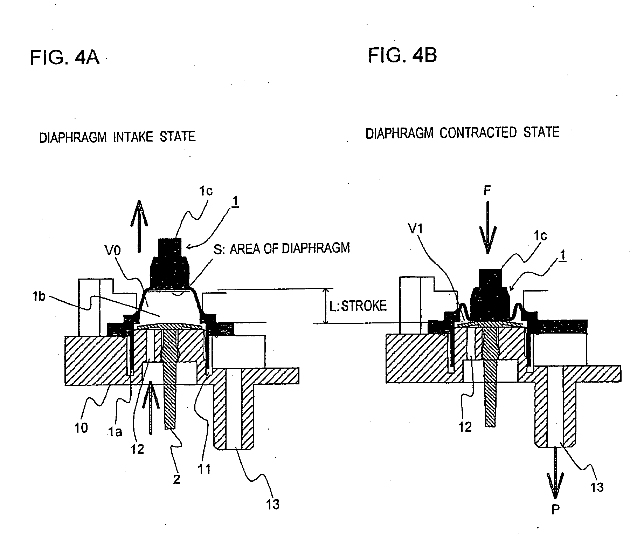

The outline of a diaphragm air pump will first be described with reference to Figs. 2, 3, and Figs. 4A and 4B. Figs. 2 and 3 are exploded perspective views of the diaphragm air pump. Figs. 4A and 4B are sectional views that depict an intake state and a contracted state of the diaphragm air pump, respectively.

An air pump A includes a diaphragm 1, a check valve 2, a housing member 10 to which a part of the diaphragm 1 is fixed, the other housing member 20, and an actuator 30 to be described later.

The diaphragm 1 includes an annular discharge valve (fitted portion) 1a for fixing a part of the diaphragm 1 to the housing member 10, a pump chamber 1b formed between the diaphragm 1 and the housing member 10 and having a volume changed by a deformation of the diaphragm 1, and an abutment portion 1c provided so that an end of the actuator 30 abuts on an upper portion of the pump chamber 1b and inputting a driving force. The diaphragm 1 is deformed when the end of the actuator 30 moving according to expansion or contraction abuts on the abutment portion 1c to press the diaphragm 1.

A material for the diaphragm 1 may be an arbitrary repeatedly deformable material. For example, one of elastic bodies including thermoplastic elastomer (TPE), nitrile rubber (NBR), fluoro rubber, chloroprene rubber (CR), ethylene propylene rubber (EPDM), etc. can be appropriately selected according to a volume of the pump and a property of a fluid which is discharged.

The housing member 10 includes an annular groove 11 to which the discharge valve 1a of the diaphragm 1 is attached, an intake portion 12 for intaking the fluid into the pump chamber 1b of the diaphragm 1 from outside, a discharge portion 13 for discharging the fluid in the pump chamber 1b to the outside, and a support hole 14 provided to support fixing the check valve 2 by a pressure difference between the outside and the pump chamber 1b. The housing member 20 fixes one end of the actuator 30.

The check valve 2 is provided between the intake portion 12 and the pump chamber 1b, and a shaft of the check valve 2 is attached to the support hole 14. The check valve 2 means a valve that is opened if a pressure of one of two regions, between which the valve is put, is higher than that of the other region, and that remains closed if the pressure of the other region is higher than that of one region.

In the air pump A, as shown in Fig. 4A, if the fluid is intaked into the pump chamber 1b from the outside by driving the actuator 30, then a gap is formed between the check valve 2 and the housing member 10, and the pump chamber 1b is filled with the fluid intaked from the intake portion 12.

Thereafter, as shown in Fig. 4B, if a contraction load F is applied to the abutment portion 1c of the diaphragm 1 by a reciprocating motion of the actuator 30, then the diaphragm 1 is deformed and the volume of the pump chamber 1b is changed. If so, the check valve 2 closes the intake portion 12, the fluid intaked into the pump chamber 1b is pressurized, the discharge valve 1a is opened, and the fluid is discharged to the outside from the discharge portion 13.

A pressure flow rate (discharge flow rate) Q (ml/min), a load F (N/cm2) necessary for contraction, and a discharge pressure P (mmHg=$1.332 \times 10-2$N/cm2) of the air pump A are calculated by the following equations (1), (2), and (3), respectively.

$$Q = \eta p \times 760/(760+P) \times V \times f \times 60 \quad \text{equation (1)}$$

$$F = F0 + (S \times P \times 1.332 \times 10-2) \quad \text{equation (2)}$$

$$P = 760 \times (V0/V1) \kappa - 760 \quad \text{equation (3)}$$

In the equations (1), (2), and (3), respective symbols denote the following.

$\eta$ p: pump efficiency

V (ml)=V0 (volume of pump chamber at atmospheric pressure) - V1 (volume of pump chamber during contraction): volume change rate

f (Hz): frequency

S (cm2): area of diaphragm

F0 (N/cm2): diaphragm deformation load (at atmospheric pressure)

$\kappa$ : adiabatic change

As can be understood from the equations (1) to (3), the discharge pressure P can be calculated by a ratio of V0 to V1, i.e., a contraction ratio, and can be controlled by changing a stroke L from a top dead center to a bottom dead center of the diaphragm 1 shown in Figs. 4A and 4B. The pressure flow rate Q can be controlled by controlling the volume change V and the frequency f as well as the discharge pressure P.

(OUTLINE OF ACTUATOR USED IN AIR PUMP)

As the actuator 30, an actuator including an elastomer or a polymer expandable or contractible when a voltage is applied thereto, to be described later, and electrodes provided to apply the voltage to the elastomer or polymer is employed in place of a motor. It is thereby possible to provide a small-sized air pump having a simple configuration.

Specifically, anElectroactivePolymer Artificial Muscle ("EPAM") is most preferably used as the expandable or contractible elastomer or polymer. The following embodiments will be described, assuming that the EPAM is employed in the actuator 30.

The actuator 30 employing the EPAM can change an expansion magnitude (stroke L) according to a magnitude of the applied voltage. Therefore, by controlling a voltage and a frequency of a signal input to drive the actuator 30, the pressure flow rate Q and the discharge pressure P can be easily changed.

Namely, specifications of the diaphragm 1 and the actuator 30 that drives the diaphragm 1 can be individually set according to necessary pressure and flow rate. Due to this, if a high discharge pressure is required at a small driving force, the area of the diaphragm 1 may be reduced or the stroke of the actuator 30 may be increased (the applied voltage may be increased) to increase the contraction ratio. If a high pressure flow rate is required, the volume of the pump chamber 1b of the actuator 30 may be increased or a driving frequency of the actuator 30 or the applied pressure to the actuator 30 may be increased.

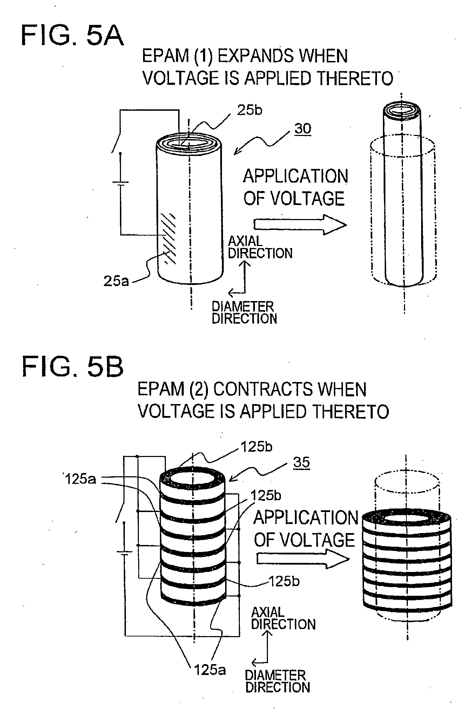

Figs. 5A and 5B are typical views that respectively depict deformation states when a voltage is applied to the actuator 30 according to the embodiments.

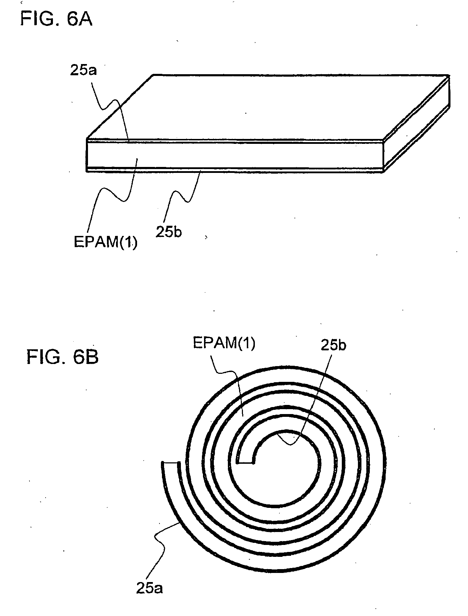

The actuator 30 according to the present embodiment is configured so that a film-like EPAM (1) having flexible electrodes 25a and 25b provided on both surfaces, respectively, as shown in Fig. 6A, is wound into a roll by one turn or a plurality of turns as shown in Fig. 6B. An insulator, not shown, is wound into a roll together with the EPAM (1) to isolate the adjacent electrodes from each other when the EPAM (1) is wound. As shown in Fig. 5A, a voltage V is applied between the electrodes 25a and 25b, whereby the actuator 30 contracts in a diameter direction and expands in an axial direction. The actuator 30 is, therefore, to expand in a vertical direction of the diaphragm 1. However, since one end of the actuator 30 is fixed to an inner wall of the housing member 20, the pump chamber 1b of the diaphragm 1 is contracted by causing the other end of the actuator 30 to press the abutment portion 1c of the diaphragm 1.

By using above-mentioned actuator 30, the air pump A according to the present embodiments enables the deformation of the diaphragm 1 from an initial (intake) state (Fig. 4A) to a contracted state (Fig. 4B) without using a complicated mechanism such as a motor or a clutch.

Further, if the application of the voltage is stopped, then the deformed actuator 30 returns to it original shape by a restoring force of a spring provided in a central portion of the actuator 30. The diaphragm 1, therefore, returns from the contracted state to the initial state.

According to another embodiment, electrodes 125a and 125b are provided on both ends in the axial direction of an EPAM (2), respectively, as shown in Fig. 5B. In another embodiment, a plurality of electrodes 125a and 125b are alternately provided between the EPMA (2). By applying the voltage V between the electrodes 125a and 125b, the actuator 35 contracts in the axial direction and expands in the diameter direction. The actuator 35 is, therefore, to contract in the vertical direction of the diaphragm 1. However, since one end of the actuator 35 is fixed to the inner wall of the housing member 20, the other end of the actuator 35 pulls up the abutment portion 1c of the diaphragm 1, whereby the fluid is intaked into the pump chamber 1b within the diaphragm 1.

By using above-mentioned actuator 35, the air pump A according another embodiment enables the deformation of the diaphragm 1 from the initial (contracted) state (Fig. 4B) to the intake state (Fig. 4A) without using the complicated mechanism such as a motor or a clutch.

Further, if the application of the voltage is stopped, then the deformed actuator 35 returns to it original shape by the restoring force of the spring provided in the central portion of the actuator 35. The diaphragm 1, therefore, returns from the intake state to the contracted state.

The air pump A according to the present embodiments can, therefore, dispense with the complicated mechanism such as a motor or a clutch as required in the conventional technique, and also with the components for converting a motion direction from the rotational motion into the reciprocating motion. Therefore, the air pump having a simple configuration can be provided. In addition, the air pump can be made small in size and light in weight, as compared with the air pump that employs the motor.

Further, since a motor driving sound is not produced, noise generated while the air pump is driven can be reduced. Besides, since there is no need to apply a current for driving the motor, the power consumption of the entire air pump can be suppressed.

Moreover, as compared with the instance of employing the disk-like electrostrictive polymer actuator for the diaphragm as described in the conventional techniques, the volume change of the pump chamber can be easily increased.

[FIRST EMBODIMENT]

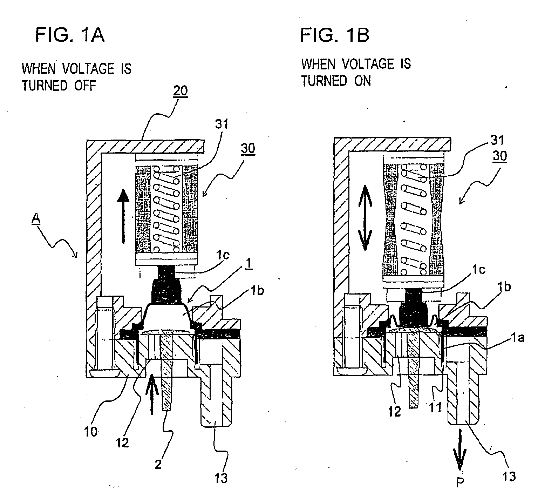

Figs. 1A and 1B are schematic sectional views that respectively depict operating states of the air pump A that employs the actuator 30 mentioned above.

As shown in Fig. 1A, if the voltage applied to the actuator 30 is turned off, the diaphragm 1 is stationary at a position at which the volume of the pump chamber 1b is a maximum volume. In this state, the pump chamber 1b and the outside are substantially equal in pressure. The fluid introduced from the intake portion 12, therefore, flows into the pump chamber 1b from the gap between the check valve 2 and the housing member 10.

Thereafter, as shown in Fig. 1B, if the voltage applied to the actuator 30 is turned on, the actuator 30 expands vertically and press the abutment portion 1c of the diaphragm 1, thereby reducing the volume of the pump chamber 1b. At this moment, the internal pressure of the pump chamber 1b is increased, so that the check valve 2 is closed and the fluid intaked from the intake portion 12 is shut off. In addition, since the pump chamber 1b is contracted, the internal pressure of the pump chamber 1b is increased and the annular discharge valve 1a of the diaphragm 1 is deformed. As a result, the fluid flows out from the gap between the discharge valve 1a and the annular groove 11 of the housing member 10 and reaches the outside through the discharge portion 13. At this time, the discharge valve 1a functions as a check valve provided between the pump chamber 1b and the discharge portion 13.

If the voltage applied to the actuator 30 is turned off again, the actuator 30 is contracted by the restoring force of the spring 31 provided in the actuator 30. As a result, the volume of the pump chamber 1b is increased and the pressure thereof is reduced. Therefore, the moment the pressure of the pump chamber 1b is reduced to be lower than the external pressure, the check valve 2 is opened and the fluid is intaked into the pump chamber 1b from the outside.

As can be seen, by repeating the states shown in Figs. 1A and 1B according to the reciprocating motion (expansion and contraction motion) of the actuator 30, the volume and the pressure of the pump chamber 1b of the diaphragm 1 can be changed. It is, therefore, possible to provide the air pump having a simple configuration without using a power transmission member such as a motor or a gear.

In other words, the air pump A according to the first embodiment can dispense with the complicated mechanism such as the motor or clutch as required in the conventional techniques, and also with the components for converting a motion direction from the rotational motion into the reciprocating motion. The air pump having a simple configuration can be, therefore, provided. In addition, the air pump can be made small in size and light in weight, as compared with the air pump that employs the motor.

Further, since a motor driving sound is not produced, noise generated while the air pump A is driven can be reduced. In addition, since the air pump A operates at a lower current than that for driving the motor, the power consumption of the entire air pump can be suppressed.

Moreover, as compared with the conventional techniques in which the disk-like electrostrictive polymer actuator is employed for the diaphragm, the volume change of the pump chamber 1b can be easily increased.

[SECOND EMBODIMENT]

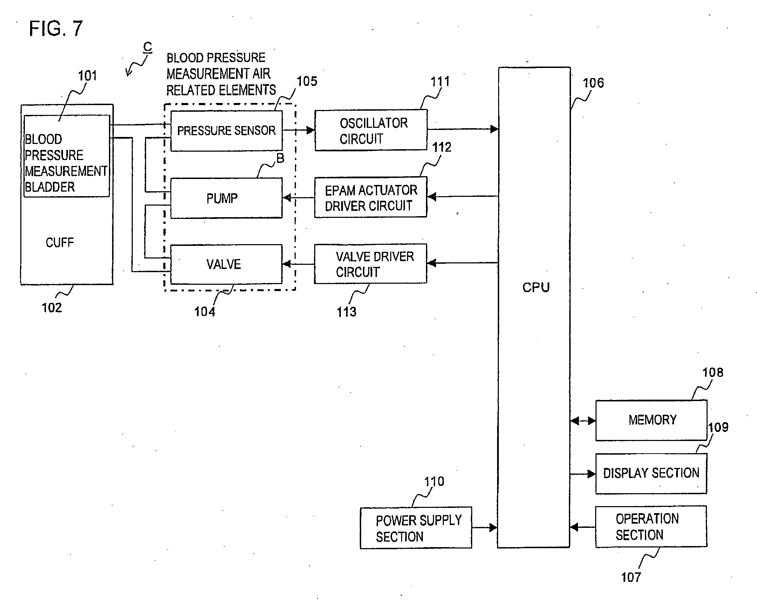

In a second embodiment, an air pump suitably used in an electronic sphygmomanometer will be described. Fig. 7 is a block diagram that depicts a hardware configuration of the electronic sphygmomanometer.

(CONFIGURATION OF SPHYGMOMANOMETER)

An electronic sphygmomanometer C includes a fluid bag (bladder) 101 wound around an upper arm (a living body) during measurement of blood pressure, a compressing and fixing cuff 102 that compresses and fixes the fluid bag 101 from surroundings of the fluid bag 101, the air pump B that supplies a fluid such as the air to the fluid bag 101 to be filled with the fluid and that pressurizes the fluid bag 101, a valve 104 that discharges the fluid within the fluid bag 101, a pressure sensor 105 that detects an internal air pressure of the fluid bag 101, a CPU 106 serving as arithmetic means for executing a processing for blood pressure measurement according to a program included in the CPU 106 based on the detected internal air pressure, an operation section 107 that makes settings during the measurement and that starts the measurement, a memory 108 that stores setting data, arithmetic data, a measurement result, etc., a display section 109 that displays a setting state, the measurement result, etc., and a power supply section 110 that supplies power to the respective constituent elements.

Further, the CPU 106 detects the internal pressure of the fluid bag 101 based on a signal output from the pressure sensor 105 and converted by an oscillator circuit 111. The CPU 106 controls a driver circuit 112 of the actuator 30 with EPAM to drive the air pump B to increase the internal pressure of the fluid bag 101 if it is necessary to apply pressure. The CPU 106 controls a valve driver circuit 113 to open the valve 104 to reduce the internal pressure of the fluid bag 101 if it is necessary to reduce pressure.

(BASIC OPERATION OF SPHYGMOMANOMETER)

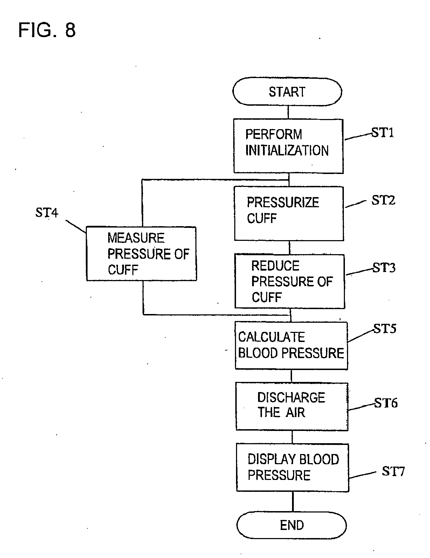

Fig. 8 is a flowchart that depicts a basic operation of the electronic sphygmomanometer to which the present invention can be suitably applied.

When power of the electronic sphygmomanometer is turned on and the electronic sphygmomanometer starts operating after the cuff 102 is wound around the upper arm (living body), initialization for resetting respective setting states of the electronic sphygmomanometer C to initial states is performed (in a step ST1).

The fluid bag 101 wound around the upper arm (living body) is pressurized up to a predetermined pressure by the air pump B (in a step ST2). At the same time, a signal indicating a change in the pressure of the fluid bag 101 detected by the pressure sensor 105 is transmitted to the CPU 106 through the oscillator circuit 111, and the measurement of the blood pressure is started in response to the signal (in a step ST4).

After the end of the pressurization, the pressure of the fluid bag 101 is gradually reduced by opening the valve 104 (in a step ST3). At the same time, a signal indicating a change in the pressure of the fluid bag 101 detected by the pressure sensor 105 is transmitted to the CPU 106 through the oscillator circuit 111, and the CPU 106 then calculates a highest blood pressure, a lowest blood pressure, and a pulse frequency (in a step ST5).

When the measurement is finished, the air within the fluid bag 101 that has compressed the upper arm is discharged from the valve 104, whereby the upper arm is unclamped (in a step ST6).

The calculated blood pressure values and the like are displayed on the display section 109 (in a step ST7), thus finishing one measurement operation cycle.

A configuration of an air pump suitably used in the electronic sphygmomanometer according to the present invention and including a control valve will be described. An instance of measuring the blood pressure of the upper arm of a human body will be described hereafter. However, the electronic sphygmomanometer is also applicable to living bodies other than the human body, and a measurement target region may be a wrist or an ankle that is a part of the living body.

(SCHEMATIC CONFIGURATION OF AIR PUMP INCLUDING SOLENOID VALVE)

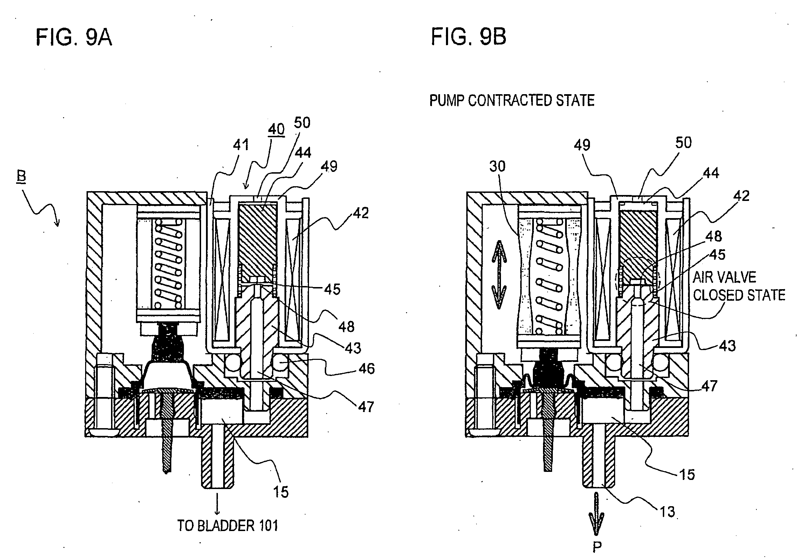

Figs. 9A and 9B are schematic sectional views that respectively depict operating states of an air pump B that is used in the sphygmomanometer that employs the actuator 30. The air pump B according to the present embodiment greatly differs from the air pump A according to the first embodiment in that the air pump B includes, as the control valve, a solenoid valve 40 in place of the valve 104. The control valve controls open and close operations of a valve in response to an external signal. The same constituent elements as those of the air pump A will not be described herein, and a configuration of the solenoid valve 40 and an operation of the air pump B during blood pressure measurement will be mainly described herein.

The solenoid valve 40 includes a frame 41, a coil 42 wound cylindrically inside the frame 41, a fixed core 43 fixedly provided in the coil 42 and serving as a core, a movable core 44 that is driven to slide by an attracting force of a magnetic flux generated by a current carried across the coil 42, an air packing 45 provided on a tip end of the movable core 44, and O rings 46 disposed between the frame 41 and the housing member 10.

The pump chamber 1b communicates with the discharge portion 13 by a passage 15 provided in the housing member 10. A shaft hole 47 that communicates with the passage 15 is formed at the center of the fixed core 43. When power is turned off or the measurement is finished, the fixed core 43 is distant from the movable core 44 as shown in Fig. 9A. In addition, the shaft hole 47 communicates with an exhaust port 50 provided in a bobbin 49 that is a member around which the coil 42 is wound, and releases the air pressurized by the fluid bag 101 to the outside.

When the blood pressure measurement is started, a current is carried across the coil 42, thereby moving the movable core 44 toward the fixed core 43 (Fig. 9B). Accordingly, the air packing 45 closes a nipple 48 of the fixed core 43, and the passage between the exhaust port 50 and the shaft hole 47 is shut off (an air valve closed state shown in Fig. 9B). At the same time, the air pump B drives the actuator 30 to repeat air intake and contraction according to the volume change of the diaphragm 1. The air is thereby supplied from the discharge portion 13 to the fluid bag 101, thus pressurizing the upper arm (refer to the step ST3 of Fig. 8).

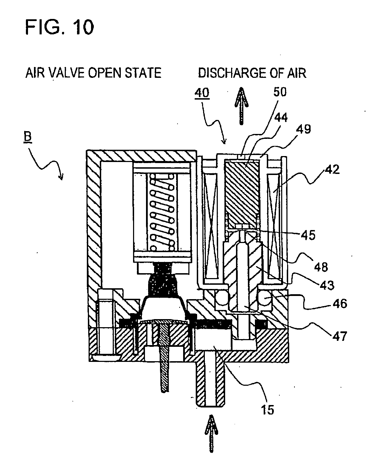

After the pressurization is finished, the current applied to the coil 42 of the solenoid valve 40 is controlled to be reduced, whereby attracting force between the movable core 44 and the fixed core 43 is reduced. A gap between the air packing 45 and the nipple 48 is controlled to be opened (refer to Fig. 10), and the pressure of the fluid in the passage 15 and in the fluid bag 101 is gradually reduced, thereby controlling pressure reduction (refer to the step ST3 of Fig. 8).

After the measurement of the blood pressure is finished, the gap between the air packing 45 and the nipple 48 is completely opened, and the air pressurized by the fluid bag 101 is released to the outside from the exhaust port 50, thereby finishing constraining the upper arm (the living body).

The air pump B according to the present embodiment can dispense with the complicated mechanism such as the motor or clutch as required in the conventional techniques, and also with the components for converting the motion direction from the rotational motion into the reciprocating motion. The air pump having a simple configuration canbe, therefore, provided. In addition, the air pump can be made small in size and light in weight, as compared with the air pump that employs the motor.

Further, since a motor driving sound is not produced, noise generated while the air pump B is driven can be reduced. In addition, since a current for driving the motor is unnecessary, the power consumption of the entire air pump can be suppressed.

Moreover, as compared with the conventional techniques in which the disk-like electrostrictive polymer actuator is employed for the diaphragm, the volume change of the pump chamber can be easily increased.

Furthermore, by employing the airpump that controls the pressure of the fluid discharged to the external fluid bag using the control valve, the small-sized living body pressurization air intake and exhaust device and the small-sized electronic sphygmomanometer each having a simple configuration can be provided.

**Claims**

1.  An air pump comprising:

an actuator including an elastomer or a polymer expandable or contractible when a voltage is applied to the elastomer or polymer, and electrodes provided to apply the voltage to the elastomer or polymer; and
a diaphragm deformed by a reciprocating motion of the actuator.

2. An air pump comprising:

an actuator including an elastomer or a polymer expandable or contractible when a voltage is applied to the elastomer or polymer, and electrodes provided to apply the voltage to the elastomer or polymer;
one or a plurality of housing members; and
a diaphragm a part of which is fixed to at least one of the housing members, wherein
one end of the actuator is fixed to the at least one of the housing members,
the diaphragm is pressurized and deformed by an other end of the actuator that moves according to an expansion or a contraction of the elastomer or polymer, and
a volume of a pump chamber formed between the diaphragm and the at least one of the housing members is changed by a deformation of the diaphragm, thereby pressurizing a fluid intaked into the pump chamber and discharging the fluid to an outside.

3. An air pump according to claim 2, wherein
the diaphragm includes an abutment portion abutting the other end of the actuator.

4. An air pump according to claim 2 or 3, wherein
a magnitude of the expansion or the contraction of the actuator is changed according to a magnitude of the voltage applied to the actuator.

5. An air pump according to any one of claims 2 to 4, wherein
the voltage applied to the actuator or a frequency of the voltage is controlled, thereby controlling a discharge flow rate and a discharge pressure of the fluid discharged from the pump chamber.

6. An air pump according to any one of claims 2 to 5, further comprising:

an intake portion for intaking the fluid into the pump chamber from the outside; and
a discharge portion for discharging the fluid from the pump chamber to the outside, wherein
check valves are provided between the intake portion and the pump chamber, and between the pump chamber and the discharge portion, respectively.

7. An air pump according to any one of claims 2 to 6, further comprising:

a control valve that controls a pressure of the fluid discharged to the outside, wherein
a passage communicating with the control valve is provided halfway from the pump chamber to the discharge portion.

8. A living body pressurization air intake and exhaust device comprising:

a fluid bag wound around a living body and filled with a fluid;
a cuff for fixing the fluid bag from surroundings of the fluid bag; and
the air pump according to any one of claims 1 to 7, for supplying the fluid to the fluid bag and pressurizing the fluid bag.

9. An electronic sphygmomanometer comprising:

a fluid bag wound around a living body and filled with a fluid such as the air;
a cuff for fixing the fluid bag from surroundings of the fluid bag;
the air pump according to any one of claims 1 to 7, for supplying the fluid to the fluid bag and pressurizing the fluid bag;
a pressure sensor that detects an internal air pressure of the fluid bag; and
arithmetic means for executing a processing for measurement of blood pressure based on the detected internal air pressure.

FIG. 1A

WHEN VOLTAGE IS TURNED OFF

FIG. 1B

WHEN VOLTAGE IS TURNED ON

FIG. 2

FIG. 3

# FIG. 4A

DIAPHRAGM INTAKE STATE

S: AREA OF DIAPHRAGM

L:STROKE

# FIG. 4B

DIAPHRAGM CONTRACTED STATE

EP 1 621 129 A1

# FIG. 5A

EPAM (1) EXPANDS WHEN
VOLTAGE IS APPLIED THERETO

25b

30

APPLICATION
OF VOLTAGE

AXIAL
DIRECTION

25a

DIAMETER
DIRECTION

# FIG. 5B

EPAM (2) CONTRACTS WHEN
VOLTAGE IS APPLIED THERETO

125b

35

125a

125b

125b

APPLICATION
OF VOLTAGE

125b

AXIAL
DIRECTION

125a

DIAMETER
DIRECTION

FIG. 6A

FIG. 6B

FIG. 7

FIG. 8

```
          ┌──────────┐
          │  START   │
          └────┬─────┘
               │
          ┌────────────┐
          │ PERFORM    │──ST1
          │ INITIALIZATION │
          └────┬───────┘
               │
          ┌────────────┐
          │ PRESSURIZE │── ST2
          │ CUFF       │
          └────┬───────┘
               │
┌──────────┐   ┌────────────┐
│ MEASURE  │   │ REDUCE     │──ST3
│ PRESSURE OF│ │ PRESSURE OF│
│ CUFF     │   │ CUFF       │
└──────────┘   └────┬───────┘
  ST4               │
          ┌────────────┐
          │ CALCULATE  │──ST5
          │ BLOOD PRESSURE │
          └────┬───────┘
               │
          ┌────────────┐
          │ DISCHARGE  │──ST6
          │ THE AIR    │
          └────┬───────┘
               │
          ┌────────────┐
          │ DISPLAY BLOOD │
          │ PRESSURE   │── ST7
          └────┬───────┘
               │
          ┌──────────┐
          │   END    │
          └──────────┘
```

FIG. 9A

FIG. 9B

PUMP CONTRACTED STATE

AIR VALVE CLOSED STATE

TO BLADDER 101

## FIG. 10

AIR VALVE OPEN STATE        DISCHARGE OF AIR

**European Patent Office**

# EUROPEAN SEARCH REPORT

Application Number

EP 05 01 5805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 369 584 A (SEIKO EPSON CORPORATION) 10 December 2003 (2003-12-10) * the whole document * ----- | 1-9 | A61B5/022 F04B43/04 |
| Y | US 6 164 933 A (TANI ET AL) 26 December 2000 (2000-12-26) * the whole document * ----- | 1-9 | |
| Y | DE 87 04 314 U1 (SIEMENS AG, 1000 BERLIN UND 8000 MUENCHEN, DE) 25 June 1987 (1987-06-25) * the whole document * ----- | 1-9 | |

| TECHNICAL FIELDS SEARCHED (IPC) |
|---|
| A61B F04B |

The present search report has been drawn up for all claims

| Place of search | Date of completion of the search | Examiner |
|---|---|---|
| Munich | 30 November 2005 | Sopelana Martínez, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 01 5805

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2005

| Patent document cited in search report | | Publication date | Patent family member(s) | | Publication date |
|---|---|---|---|---|---|
| EP 1369584 | A | 10-12-2003 | CN | 1469046 A | 21-01-2004 |
| | | | US | 2004013548 A1 | 22-01-2004 |
| US 6164933 | A | 26-12-2000 | DE | 19918694 A1 | 04-11-1999 |
| DE 8704314 | U1 | 25-06-1987 | NONE | | |

EPO FORM P0459

For more details about this annex : see Official Journal of the European Patent Office, No. 12/82